# EUROPEAN PATENT SPECIFICATION

(11) **EP 2 316 530 B1**
(45) Date of publication and mention of the grant of the patent: **13.08.2014**
(21) Application number: 10184846.3
(22) Date of filing: 12.05.2005
(51) Int. Cl.: A61P 25/24, A61K 31/00, A61K 31/20

(54) **TRIGLYCERIDES, FATTY ACIDS, ALCOHOLS AS CNS MODULATORS**
TRIGLYCERIDE, FETTSÄUREN UND ALKOHOLE ALS CNS MODULATOREN
TRIGLYCERIDES, ACIDES GRAS ET ALCOHOLS COMME MODULATEURS DU CNS

(30) Priority: 12.05.2004 US 570137 P; 20.09.2004 GB 0420856
(43) Date of publication of application: 04.05.2011
(62) Divisional of application: 05742109.1
(73) Proprietor: BTG International Limited, London EC4M 7RD (GB)
(72) Inventor: Dimpfel, Wilfred, D - 35578, Wetzlar (DE); Greenwood, David, London EC4M7RD (GB)
(74) Representative: Dolan, Anthony Patrick

(56) References cited:
- FR-A- 2 531 859
- JP-A- 2003 306 430
- US-A- 3 284 298
- US-B1- 6 579 543
- DATABASE WPI Week 199834 Thomson Scientific, London, GB; AN 1998-393388 XP002629018, & JP 10 158166 A (TAISHO PHARM CO LTD) 16 June 1998 (1998-06-16)
- KOLYUTSKAYA O D ET AL: "Dependence of the clinical picture of anesthesia on the concentration of oxybutyrate in the blood", KHIRURGIYA 1972, vol. 48, no. 9, 1972, pages 51-53, XP8134496, ISSN: 0023-1207

## Description

The present invention relates to compounds and compositions that have the effect of modulating mammalian central nervous system activity such as to increase tolerance with respect to pain stimuli. The present invention further provides the treatment of a patient in need of therapy for pain comprising administering to said patient a therapeutically effective amount of a compound or composition of the invention. A still further aspect of the present invention provides the enhancement of tolerance of pain of a mammalian subject comprising administering an amount of the compound or composition of the invention sufficient to effect said enhancement.

It is known that both acute and chronic neurodegenerative states in mammals, eg. man, can be treated by inducing ketosis. Such ketosis can be provided by restriction of diet, eg by starvation or exclusion of carbohydrate, or by administration of ketogenic materials, such as triglycerides, free fatty acids, alcohols (eg butan-1, 3-diol), acetoacetate and (R)-3-hydroxybutyrate and their conjugates with each other and further moieties, eg. esters and polymers of these. Ketogenic materials thus produce a physiologically acceptable ketosis when administered to a patient.

Further therapeutic indications for the application of ketosis include epilepsy, diabetes, dystrophies and mitochondrial disorders. In the case of epilepsy ketogenic diet has been applied in treatment of intractable seizures with some success for many years, although the mechanism by which the seizure suppression is achieved remains uncertain.

The present inventors have been studying the mode of action of ketogenic materials in CNS injury and particularly have studied whole mammalian brain electrical activity with a view to understanding more completely its overall effect on functioning brain. Surprisingly, they have now found that completely unanticipated changes in brain electrical activity are induced by ketosis such that it is evident that mood, cognition and tolerance of pain are each affected in a positive fashion.

Analysis of brain field potentials ("Tele-Stereo-EEG") has been proven to be a very sensitive tool for the characterization of drug effects on the central nervous system (Dimpfel et al., 1986). After administration of a centrally active drug, quantitative changes in the brain field potentials can be considered as a characteristic fingerprint of that particular drug. "Fingerprints" of more than 100 compounds have been obtained including 8 established drug categories, e.g. analgesics, antidepressants, neuroleptics, stimulants, tranquilizers, sedatives and narcotics. Different dosages of the same drug cause quantitative changes in electrical power. This methodology can therefore also demonstrate possible dose response relationships. Direct comparison with specific reference drugs, or by discriminant analysis with reference to an extensive fingerprint database, permits the detection of any possible similarities with established drugs. In general, "fingerprints" show prominent differences for drugs prescribed for different indications and are similar for drugs with similar indication (Dimpfel 2003). Furthermore, the pattern of EEG changes in the rat is a useful tool in predicting possible changes in the EEG power spectrum in humans.

Applying this technique to ketosis, particularly that induced by direct administration of (R)-3-hydroxybutyrate sodium salt, the present inventors have been able to clearly show whole brain effects consistent with the aforesaid anti-depressant, cognitive and analgesic activities.

Thus in a first aspect of the present invention there is provided the treatment of a subject in need of therapy for pain comprising administering to said subject an amount of a ketogenic material sufficient to produce a ketosis in the subject sufficient to provide analgesic effect.

The ketosis produced is preferably a state in which levels of one or both of acetoacetate and (R)-3-hydroxybutyrate concentrations in the blood of the subject are raised. Preferably the total concentration of these 'ketone bodies' in the blood is elevated above the normal fed levels to between 0.1 and 30mM, more preferably to between 0.3 and 15mum, still more preferably to between 0.5 and 10mM and most preferably to between 3 and 8mM. For the purpose of maximising levels of such compounds in the CNS it is desirable to saturate the transporter through which (R)-3-hydroxybutyrate crosses the blood brain barrier: this occurring at between 3 and 5mM.

In its broadest interpretation, the ketogenic material may be any of those used in the treatment of refractory epilepsy falling within the claims, such as creams and fats falling within the claims combined with low carbohydrate and possibly high protein eg.as set out in US 6,207,856 (Veech). However, in order to avoid undesirable consequences of such diets preferred materials are selected from acetoacetate, (R)-3-hydroxybutyrate, salts, esters and oligomers of these. Other acceptable materials are metabolic precursors of ketones namely (R)-1, 3-butandiol.

Particular materials are known from the following references as set out in Table 1 below. Doses and formats are as described in the documents identified in the table. Typically the amount of ketogenic material required can be determined by measuring blood levels directly using a meter such as the Medisense Precision Extra (MedisenseInc, 4A Crosby Drive Bedford, MA 01730); BioScanner 2000 (formerly called the MTM BioScanner 1000) from Polymer Technology Systems Inc. Indianapolis, Indiana. In this manner the amount of ketosis derived from a set dose may be ascertained, and that dose iterated to suit the individual.

Typical dose ranges for example might be in the range 5 to 5000mg/kg body weight, particularly for an (R)-3-hydroxybuytrate containing material such as oligomeric (R)-3-hydroxybuytrate or its esters with, eg, glycerol or (R)-butan-1,3-diol, more preferably 30 to 2000mg/kg body weight, most preferably 50 to 1000mg/kg body weight per day. Doses are conveniently given with meals when orally administered, conveniently before or at the same time as such meals. Regular blood levels are more readily attained by dosing three or four times a day.

In a second aspect of the present invention there is provided the use of a ketogenic material for the manufacture of a medicament for the treatment of pain.

Again, suitable ketogenic materials are as described for the first aspect of the invention and as exemplified in Table 1.

A third aspect of the present invention provides a pharmaceutical composition for treating pain comprising as active ingredient a ketogenic material falling wilthin the claims. The composition preferably includes diluent, excipient and/or carrier materials.

**TABLE 1**

| Material | Type | Reference |
|---|---|---|
| Sodium (R)-3-hydroxybutyrate | Salt | US 4579955 US 4771074 |
| (R)-1, 3-butandiol | Metabolic precursor | Gueldry al (1994) Metabolic Brain Disease Vol 9 No2 |
| Acetoacetylbutandiol | Metabolic precursor | US 4997976 US 5126373 |
| Dimer and trimer BHB | Metabolic precursor | JP 5009185 JP 2885261 |
| Acetoacetyltri-3HB | Metabolic precursor | US 6207856 |
| Mid chain tricglyceride | Metabolic precursor | WO 01/82928 |
| Triolide | Metabolic precursor | WO 00/15216 WO 00/04895 |
| BHB-triglyceride | Metabolic precursor | US 5420335 US 6306828 |
| BHB multimers | Metabolic precursor | WO 00/14985 |

The present invention will now be described by way of the following Examples and Figures.

### FIGURES

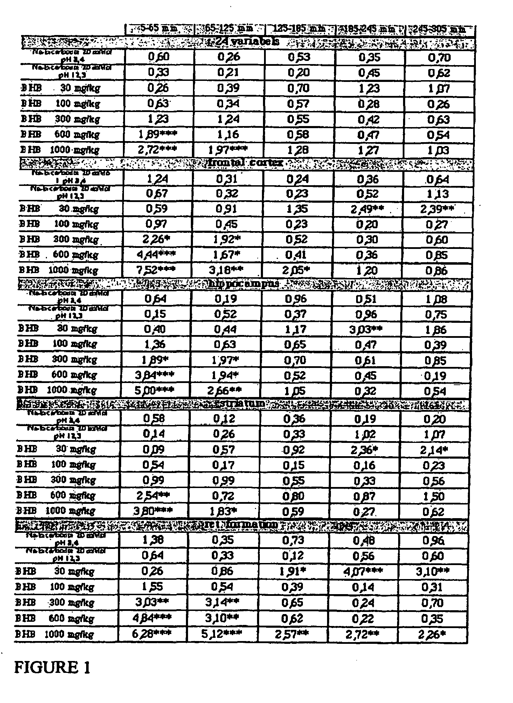

Figure 1: Approximate F - statistics during several time periods after s.c. single dose application of BHB:30; 100; 300, 600 and 1000 mg/kg body weight) and Na-bicarbonate 20mMol pH 8,4 and12,5. Variance/co-variance was estimated on the basis of 88 groups from part of our database of reference drugs with a total of 674 experiments carried out under identical conditions. Variables: frequency range - brain region *F > 1,64 corresponds to p < 0.1 and **F > 2,10 corresponds to p < 0.05 and ***F > 2,80 corresponds to p < 0.01. For evaluation of 24 variables: *F > 1,33 corresponds to p < 0.1 and **F > 1,52 corresponds to p < 0.05 and ***F > 1,79 corresponds to p < 0.01.number of experiments:n=11 (30 mg/kg) n=12 (100 mg/kg); n=12 (300 mg/kg); n=11 (600 mg/kg); n=11 (1000 mg/kg) and Na-bicarbonate 20 mMol n=11(pH8,4);n=11(pH12,5).
Figure 2: **Action of vehicle** (n=13) on the electrical power of four rat brain areas. Time - dependent changes (percentage change of pre-drug values) in EEG spectral patterns (60 min each) during 300 min after i.p. single-dose application.
   Definition of frequency ranges: delta (1.25-4.5 Hz, red), theta (4.75-6.75 Hz, orange), alphal (7.00-9.50 Hz, yellow), alpha2 (9.75-12.50 Hz, green), betal (12.75-18.50 Hz, light blue), beta2 (18.75-35.00 Hz, dark blue).
Figure 3: **Action of Na-bicarbonate 20mMol pH 8,4** (n=11) on the electrical power of four rat brain areas. Time - dependent changes (percentage change of pre-drug values) in EEG spectral patterns (60 min each) during 300 min after i.p. single-dose application. Definition of frequency ranges see Fig. 2
Figure 4: **Action of Na-bicarbonate 20mMol pH 12,5** (n=12) on the electrical power of four rat brain areas. Time - dependent changes (percentage change of pre-drug values) in EEG spectral patterns (60 min each) during 300 min after i.p. single-dose application. Definition of frequency ranges see Fig. 2.
Figure 5: **Action of BHB 30 mg/kg** (n=11) on the electrical power of four rat brain areas. Time - dependent changes (percentage change of pre-drug values) in EEG spectral patterns (60 min each) during 300 min after i.p.. single-dose application. Definition of frequency ranges see Fig. 2.
Figure 6: **Action of BHB 100 mg/kg** (n=12) on the electrical power of four rat brain areas. Time - dependent changes (percentage change of pre-drug values) in EEG spectral patterns (60 min each) during 300 min after i.p.. single-dose application. Definition of frequency ranges see Fig. 2.
Figure 7: **Action of BHB 300 mg/kg** (n=12) on the electrical power of four rat brain areas. Time - dependent changes (percentage change of pre-drug values) in EEG spectral patterns (60 min each) during 300 min after i.p.. single-dose application. Definition of frequency ranges see Fig. 2.
Figure 8: **Action of BHB 600 mg/kg** (n=11) on the electrical power of four rat brain areas. Time - dependent changes (percentage change of pre-drug values) in EEG spectral patterns (60 min each) during 300 min after i.p.. single-dose application. Definition of frequency ranges see Fig. 2.
Figure 9: **Action of BHB 1000 mg/kg** (n=11) on the electrical power of four rat brain areas. Time - dependent changes (percentage change of pre-drug values) in EEG spectral patterns (60 min each) during 300 min after i.p.. single-dose application. Definition of frequency ranges see Fig. 2.
Figure 10: **Dose-relationship of BHB** (range 30 - 1000 mg/kg); Vehicle and Na-bicarbonate (20mMol pH8,4 and 12,5) acting on the electrical power of four rat brain areas in comparison to saline within the t**ime period 5-65** min. Definition of frequency ranges see Fig. 2.
Figure 11: **Dose-relationship of BHB** (range 30 - 1000 mg/kg); Vehicle and Na-bicarbonate(20mMol pH8,4 and 12,5) acting on the electrical power of four rat brain areas in comparison to saline within the **time period 65 - 125 min.**
Definition of frequency ranges see Fig. 2.

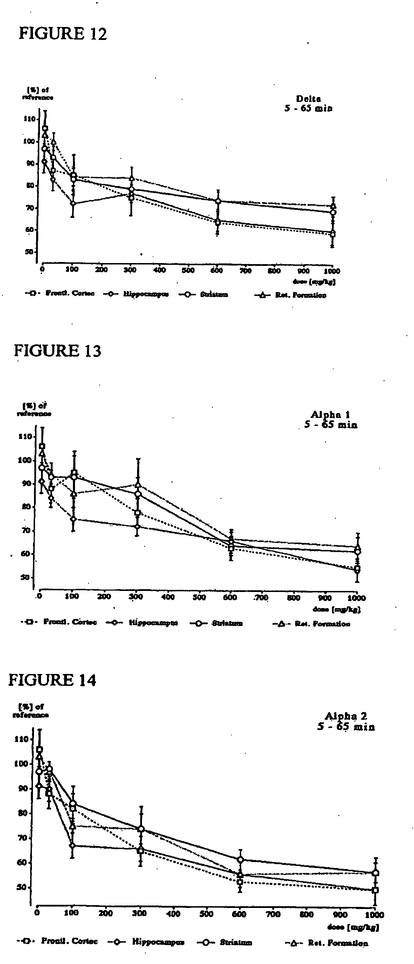

Figure 12: **Dose-relationship of BHB** (range 0 - 1000 mg/kg); acting on the electrical power of four rat brain areas in comparison to saline within the **time period 5 - 65 min Selected frequency band: Delta**
Figure 13: **Dose-relationship of BHB** (range 0 - 1000 mg/kg); acting on the electrical power of four rat brain areas in comparison to saline within the **time period 5 - 65 min Selected frequency band: alpha 1**
Figure 14: **Dose-relationship of BHB** (range 0 - 1000 mg/kg); acting on the electrical power of four rat brain areas in comparison to saline within the **time period 5 - 65 min Selected frequency band: alpha2**

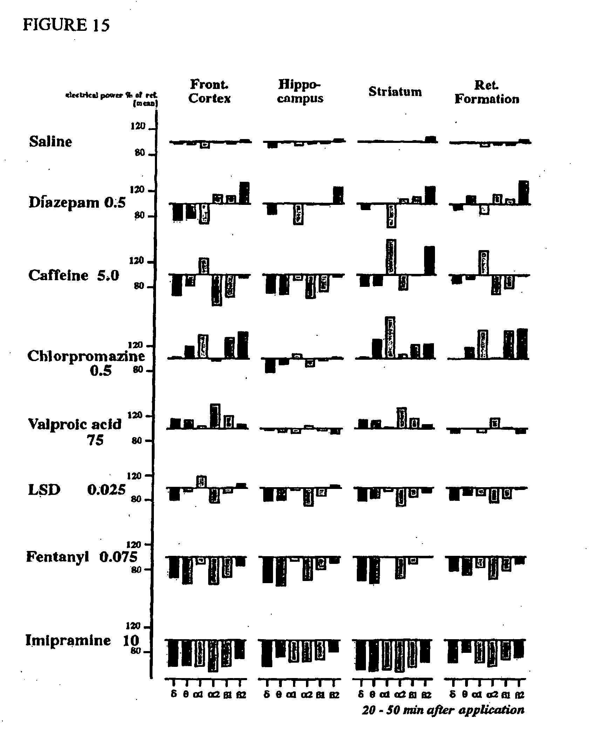

Figure 15: **Quantitative EEG finger prints** (EEG frequency pattern) of standard drugs (diazepam (0.5 mg/kg; n=6), caffeine (5 mg/kg; n=6), chlorpromazine (0.5 mg/kg; n=6), LSD (0.025 mg/kg; n=6), fentanyl (0.075 mg/kg; n=6), imipramine (10 mg/kg; n = 5), valproic acid (75 mg/kg; n=8) and Saline (0.9% NaCl₂; n=12)) after single-dose application during 20th to 50th min. Definition of frequency ranges see Fig. 1.

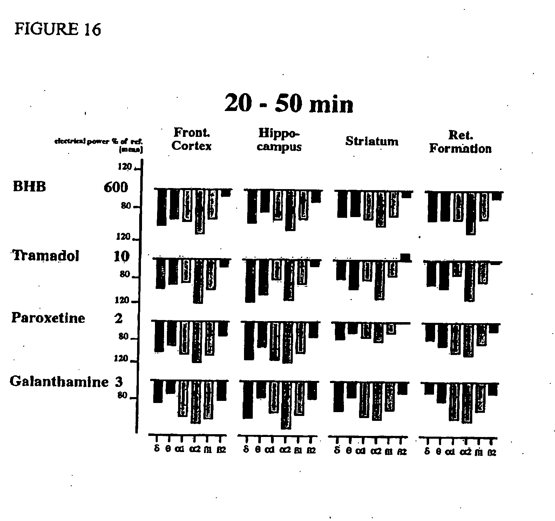

Figure 16: Comparison of the effect of BHB to several reference drugs with similar profile.

### EXPERIMENTAL EXAMPLES

Five intraperitoneal doses (30 mg/kg, 100 mg/kg, 300 mg/kg, 600 mg/kg and 1000 mg/kg body weight) of sodium (R)-3- hydroxybutyrate (BHB) were investigated using the "Tele-Stereo-EEG" animal model consisting of continuous recording of intracerebral field potentials in the freely moving rat. No effects could be measured using 1ml/kg of a solution containing 20mM of Na-bicarbonate (pH 8.4 or pH 12.5) for control purposes. Clear-cut dose and time dependent changes (decrease of electrical power in the delta, theta, alpha and betal range) were consistently observed for 2 to 3 hours after application of 100 mg/kg or more. Most marked changes were observed within the alpha2 range. The changes were statistically highly significant at dose levels of 300 - 1000 mg/kg. The pattern of changes observed with BHB are reminiscent of previously reported EEG effects seen after the administration of certain known drugs possessing, eg, cognition enhancing, antidepressant and analgesic properties.

### Method and Materials

Adult Fisher rats (4-6 month of age and day - night converted, weight about 400 g) were implanted with 4 bipolar concentric steel electrodes using a stereotactic surgical procedure (Paxinos and Watson, 1982). All four electrodes were placed 3 mm lateral within the left hemisphere. Anterior coordinates were 12.2, 5.7, 9.7 and 3.7 mm for frontal cortex, hippocampus, striatum and reticular formation, respectively. A baseplate carrying the electrodes and a 5-pin-plug was fixed to the skull by dental cement attached to 3 steel screws fixed into the skull. Animals were given two weeks for recovery from the surgical procedure. Experiments were performed in compliance with the German Health Authority Guidelines and with local authority approval. EEG signals were recorded from frontal cortex, hippocampus, striatum and reticular formation and were amplified and processed as described previously (see Dimpfel et al., 1986). After automatic artefact rejection, signals were collected in sweeps of 4 s duration and submitted to Fast Fourier transformation. The resulting electrical power spectra were divided into 6 frequency ranges: delta (0.8 - 4.5 Hz); theta (4.75 - 6.75 Hz); alphal (7.00 - 9.50 Hz); alpha2 (9.75 - 12.50 Hz); beta (12.75 - 18.50 Hz); beta2 (18.75 - 35.00 Hz). Spectra were averaged in steps of 3 minutes each and displayed on-line. In an off-line procedure spectra were averaged to give 15 minute or longer periods for further statistical analysis.

Five dosages of BHB (30, 100, 300, 600 and 1000 mg/kg body weight) (supplied by Solvias AG, CH 4002 Basel, Switzerland, batch No: SO-1058.047.1.120) and a vehicle control (0.9% w/v saline) were administered intraperitoneously to a group of 12 animals using a crossover design with at least 3 drug holidays in between the applications. Additional controls consisted of a 1 ml/kg of a solution containing 20 mM of Na-bicarbonate (adjusted to 8.4 and pH 12.5 obtained by titration with 1N NaOH) were tested to ascertain possible effects due to the alkaline pH of the BHB solution. After a pre-drug period of 45 minutes for baseline recording, drug effects were observed continuously for 300 minutes. Changes of electrical power (µV2/W) are expressed as % of the 45 min. pre-drug values. Multivariate statistics were calculated according to Ahrens and Läuter (1974).

### Results

### Normal saline and sodium bicarbonate controls

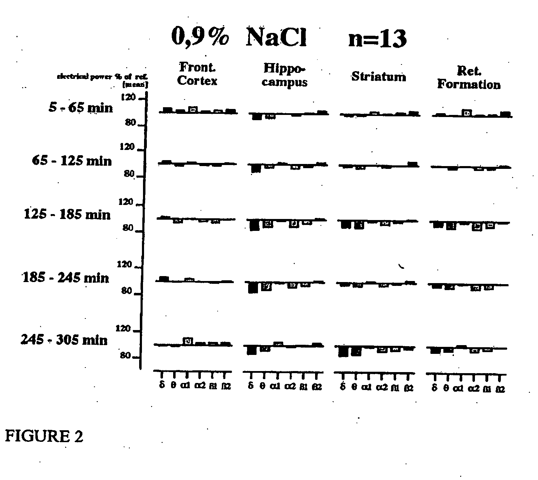

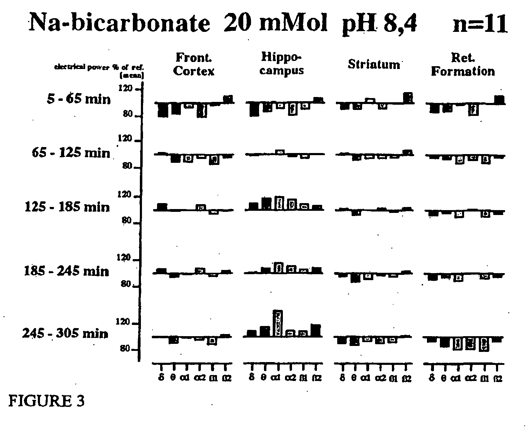

Intraperitoneal administration of 0.9% w/v saline caused only minor insignificant changes in the EEG power spectrum in comparison to the predrug values (Fig. 1). Likewise i.p. injection of 1 ml/kg of a solution containing Na-bicarbonate (pH 8.4 or 12.5) had no significant effects (Fig. 2 and 3).

### (3R)-sodium hydroxybutyrate (BHB)

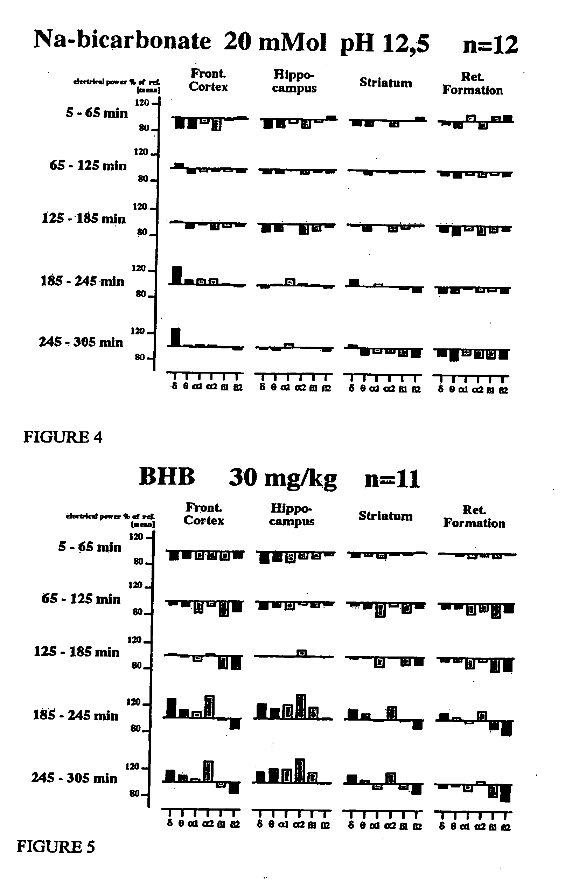

BHB (30 mg/kg body weight) Administration of BHB 30 mg/kg had no statistically significant effects on EEG frequencies relative to the saline control and was also indistinguishable from Na-bicarbonate (Fig. 4).

BHB (100 mg/kg body weight) Administration of this higher dosage of BHB resulted in frequency changes especially within the hippocampus and somewhat less within the reticular formation. All regions showed a decrease of electrical power mainly with regard to alpha2 and to a lesser extent with regard to delta frequencies. In the hippocampus theta, alphal and betal power also decreased (Fig. 5). The effects lasted for 1-2 hours only. However, these changes were not statistically significant. (Table. 1).

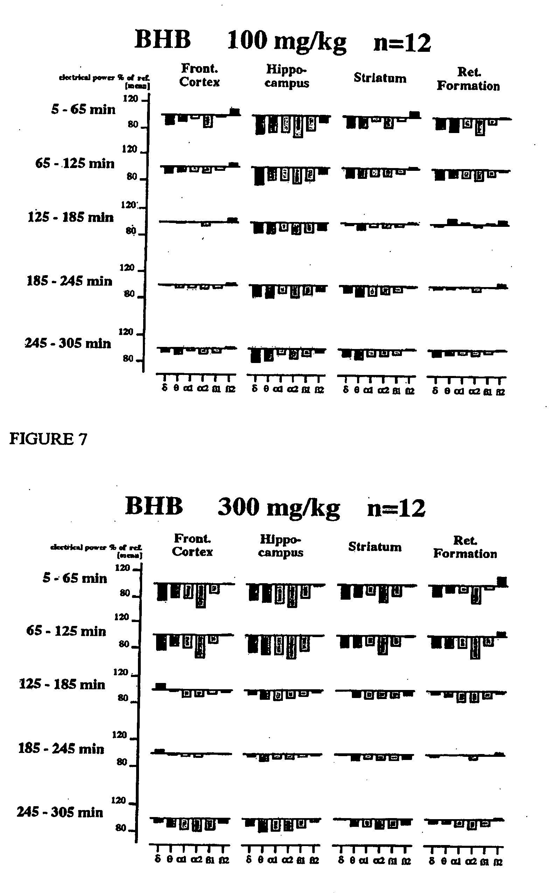

BHB (300 mg/kg body weight) BHB 300 mg/kg i.p. produced a consistent pattern of frequency changes characterized by decreases in alpha2 power throughout all brain regions. In addition, delta power changed throughout all regions but to a lesser degree. The pattern of changes (Fig. 6) lasted for exactly two hours. The changes were statistically significant for the frontal cortex, hippocampus and reticular formation, but not for the striatum (Tab. 1).

BHB (600 mg/kg body weight) BHB 600 mg/kg i.p. produced a similar pattern of change seen after 300 mg/kg. The effects generally lasted for 2 hours except for the reticular formation, where decreases of power persisted throughout the third hour (Fig. 7). The results were statistically highly significant, including the first hour within the striatum. Considering all 24 variables (6 frequencies at all four brain areas) the overall effect also became statistically significant (Tab. 1).

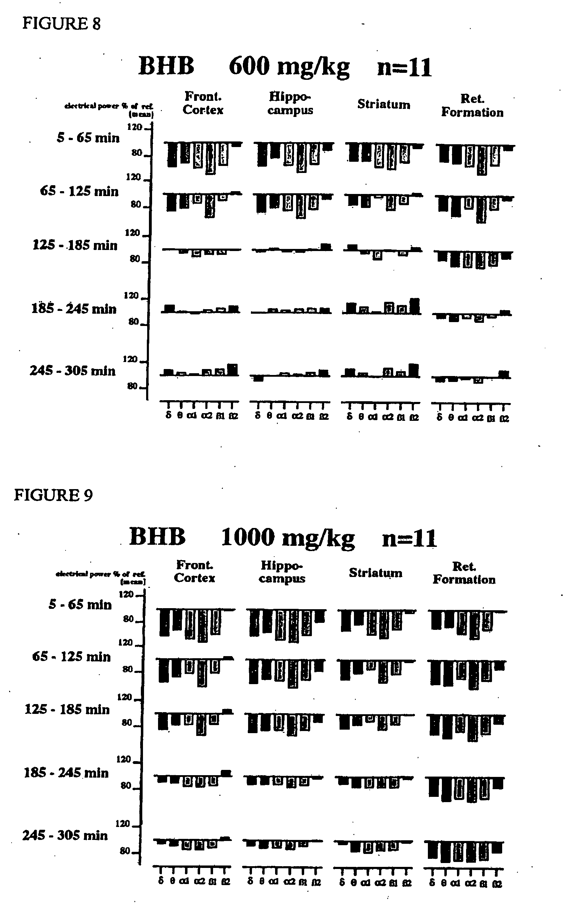

BHB (1000 mg/kg body weight) Administration of 1000 mg/kg induced an identical pattern of change but with more prominent decreases of power lasting into the third hour and, with respect to the reticular formation, throughout the total experimental time of 5 hours (Fig. 8). Again these changes were statistically highly significant, even for the 5th hour within the reticular formation (Figure. 1).

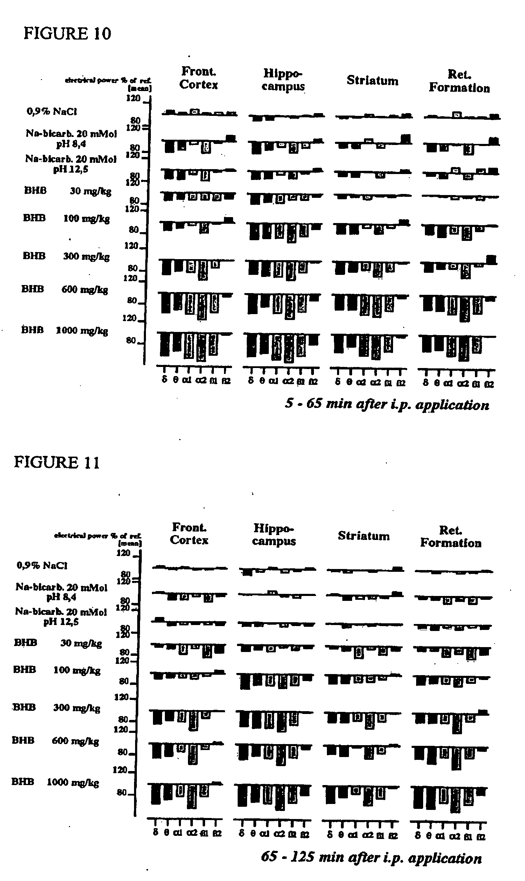

In summary, clearly dose and time dependent statistically significant changes could be observed after the administration of beta-hydroxybutyrate within a dose range of 300 to 1000 mg/kg i.p. (Fig. 9). Dose response relationships for particular frequency bands (delta, alphal and alpha2) are shown in Fig. 10a-c.

Single intraperitoneal injection of BHB within the dose range 100 to 1000 mg/kg induces clear dose related changes in the EEG power spectrum in the freely moving rat. These changes are statistically significant at 300 mg/kg in comparison with vehicle in the cortex, hippocampus and reticular formation, and also at higher dosage within the striatum (Table 1). The effects persist for 2 to 3 hours. Observed changes affect all frequencies, except for the beta2 range, but major effects are seen in the delta and alpha2 frequencies.

With regard to the specific frequency changes observed, it is known from previous studies that delta activity changes predominantly after treatment with drugs, affecting the cholinergic system, e.g. scopolamine (increase of power) or physostigmine (decrease followed by late increase due to presynaptic control of release or as rebound). Theta activity increases in response to drugs, e.g., clonidine, which down regulates the activity of the norepinephrine system, which arises in the locus coeruleus, by interacting with the presynaptic adrenergic alpha2 receptor (Dimpfel and Schober, 2001). An increase in theta activity may therefore be interpreted as a cessation of central norepinephrine transmission, a decrease signalizing arousal effects. Alpha1 activity can be modulated by drugs acting at the central serotonergic system (unpublished results). Increases of alpha1 activity often accompany relaxation whereas, in contrast, decreases signify an elevated attentional state. Furthermore, alpha2 frequencies can be influenced by drugs acting on the dopaminergic system. This has been illustrated by the studies with L-dopa, amphetamine or dopaminergic agonists e.g. SKF 393 (Dimpfel et al., 1987). Decreases of alpha2 activity, in general, are consistent with an increased state of arousal.

Since no single neurotransmitter is responsible for behaviour, the relationship or balance between these frequencies seems to be important for the psychophysiological state of the brain. In order to exemplify this, a number of drugs with known clinical indications is illustrated in Fig. 8.

The observed differences with respect to the electrical changes induced by various drugs has led us to the hypothesis that the balance of neurotransmitter action is reflected in changes of frequency content of the field potentials, i.e. the "electrical fingerprint". Therefore different drugs used for the same indication might be expected to induce similar changes of electrical activity of the brain. Indeed, this has been shown to be the case of antidepressant drugs (Dimpfel et al., 1988) and neuroleptics (Dimpfel et al., 1992) as well as other drug categories (Dimpfel, 2003).

According to this hypothesis - based on more than 30, 000 hours of recording - BHB at a lower dosage exhibits a similarity to the "electrical fingerprints" of the acetylcholine esterase inhibitor galanthamine and also the antidepressant paroxetine (3 mg/kg and 2 mg/kg , respectively; Fig. 9). It thus classifies into pharmacological groups possessing cognition enhancing and mood elevating properties, although paroxetine also has analgesic effects. In addition, the profile after higher dosages of BHB is reminiscent of the effects as observed after the administration of tramadol (10 mg/kg), another drug with analgesic properties. (Fig. 9).

The statistical differentiation of drug action is also possible using the mathematical tool of discriminant analysis. Having 6 frequency ranges and 4 different brain areas the calculations are performed with 24 variables. The results for one time period are shown in Fig. 9. Note that in addition to the 2 projection axes, results from the third to fifth discriminant function are depicted by using an additive colour mixture (similar to that used in colour TV). Thus not only is a two dimensional projection is used for classification of the EEG "fingerprint" but also the colour. This analysis of the EEG effects of BHB also places it in close proximity to paroxetine and tramadol. Thus, a similar cognition enhancing antidepressive and analgesic action of BHB might be expected in humans.

In summary, BHB has consistent effects on the conscious rat EEG fingerprint within a dose range of 100 to 1000 mg/kg body weight i.p. The overall pattern of the change in the EEG power spectrum has similarities to cognition enhancing / antidepressant and certain analgesic drugs.

### References

Ahrens H, Läuter J (1974). Mehrdimensionale Varianzanalyse. Akademie-Verlag, Berlin.
Dimpfel W, Spüler M, Nickel B (1986). Radioelectroencephalography (Tele-Stereo-EEG) in the rat as a pharmacological model to differentiate the central action of flupirtine from that of opiates, diazepam and phenobarbital. Neuropsychobiology 16: 163 - 168.
Dimpfel W, Spüler M, Koch R, Schatton W (1987). Radioelectroencephalographic comparison of memantine with drugs acting on the dopaminergic transmission in the freely moving rat. Neuropsychobiology 18: 212 - 218.
Dimpfel W, Spüler M, Borbe HO (1988). Monitoring of the effects of antidepressant drugs in the freely moving rat by radioelectroecephalography (Tele-Stereo-EEG)Neurobiology 19: 116-120.
Dimpfel W, Spüler M, Wessel K (1992). Different neurleptics show common dose and time dependent effects in quantitative field potential analysis in freely moving rats. Psychopharmacology 107: 195-202
Dimpfel W, Schober F(2001). Norepinephrine, EEG theta waves and sedation. Brain Pharmacology 1: 89-97.
Dimpfel W(2003). Preclinical data base of pharmaco-specific rat EEG fingerprints (Tele-Stereo-EEG). Eur J Med Res 8: 199-207
Paxinos G, Watson C (1982). The rat brain in stereotactic coordinates, Academic Press, New York.
Suzuki M, Suzuki M, Sato K, Dohi S, Sato T, Matsuura A, Hiraide A (2001) Effect of beta-hydroxybutyrate, a cerebral function improving agent, on cerebral hypoxia, anoxia and ischemia in mice and rats. Jpn J Pharmacol. 87: 143-50
Suzuki M, Suzuki M, Kitamura Y, Mori S, Sato K, Dohi S, Sato T, Matsuura A, Hiraide A (2002) Beta-hydroxybutyrate, a cerebral function improving agent, protects rat brain against ischemic damage caused by permanent and transient focal cerebral ischemia. Jpn J Pharmacol. 89: 36-43

## Claims

1. A ketogenic material selected from the group consisting of (R)-3-hydroxybutyrate, its salts, oligomers of (R)-3-hydroxybutyrate and esters of (R)-3-hydroxybutyrate with glycerol or (R)-1,3-butandiol for the treatment of pain wherein the treatment is by oral administration of said material at a dose of 5 to 5000mg/kg body weight per day.

2. A material for use as claimed in Claim 1 **characterised in that** the treatment is for producing a ketosis such that the total concentration of acetoacetate and (R)-3-hydroxybutyrate in the blood of the treated subject is raised to between 0.1 and 30mM.

3. A material for use as claimed in Claim 2 **characterised in that** the total concentration of acetoacetate and (R)-3-hydroxybutyrate in the blood is between 0.5 and 15mM.

4. A material for use as claimed in Claim 2 **characterised in that** the total concentration of acetoacetate and (R)-3-hydroxybutyrate in the blood is raised to between 1 and 10mM.

5. A material for use as claimed in Claim 2 **characterised in that** the total concentration of acetoacetate and (R)-3-hydroxybutyrafe in the blood is raised to between 3 and 8mM.

6. A material for use as claimed in any one of the preceding claims **characterised in that** it is in the form of a pharmaceutical composition, comprising the material as active ingredient, together with diluents, excipient and /or carrier materials.

## Patentansprüche

1. Ketogenes Material, das aus der Gruppe selektiert wurde, die aus (R)-3-Hydroxybutyrat, seinen Salzen, Oligomeren von (R)-3-Hydroxybutyrat und Estern von (R)-3-Hydroxybutyrat mit Glycerol oder (R)-1,3-Butandiol zur Schmerzbehandlung besteht, wobei die Behandlung durch orale Verabreichung des besagten Materials mit einer Dosis von 5 bis 5000 mg/kg Körpergewicht pro Tag geschieht.

2. Material zur Verwendung wie in Anspruch 1 beansprucht, **dadurch gekennzeichnet, dass** die Behandlung zur Erzeugung einer Ketose geschieht, derartig, dass die Gesamtkonzentration von Acetoacetat und (R)-3-Hydroxybutyrat im Blut der behandelten Zielperson auf zwischen 0,1 und 30 mM erhöht wird.

3. Material zur Verwendung wie in Anspruch 2 beansprucht, **dadurch gekennzeichnet, dass** die Gesamtkonzentration von Acetoacetat und (R)-3-Hydroxybutyrat im Blut zwischen 0,5 und 15 mM beträgt.

4. Material zur Verwendung wie in Anspruch 2 beansprucht, **dadurch gekennzeichnet, dass** die Gesamtkonzentration von Acetoacetat und (R)-3-Hydroxybutyrat im Blut auf zwischen 1 und 10 mM erhöht wird.

5. Material zur Verwendung wie in Anspruch 2 beansprucht, **dadurch gekennzeichnet, dass** die Gesamtkonzentration von Acetoacetat und (R)-3-Hydroxybutyrat im Blut zwischen 3 und 8 mM erhöht wird.

6. Material zur Verwendung wie in einem beliebigen der vorangehenden Ansprüche beansprucht, **dadurch gekennzeichnet, dass** es in Form von pharmazeutischer Zusammensetzung ist, die das Material als Wirkstoff, zusammen mit Verdünnungsmitteln, Bindemitteln und/oder Trägermaterialien umfasst.

## Revendications

1. Composé cétogène choisi parmi le groupe constitué par le (R)-3-hydroxybutyrate, ses sels, des oligomères de (R)-3-hydroxybutyrate et des esters de (R)-3-hydroxybutyrate avec le glycérol ou le (R3)-1,3-butanediol pour le traitement de la douleur, le traitement de la douleur s'effectuant par administration orale dudit composé à une dose de 5 à 5 000 mg/kg de poids corporel par jour.

2. Composé pour utilisation telle que revendiquée dans la Revendication 1, **caractérisé en ce que** le traitement vise à produire une cétose de manière à ce que la concentration totale d'acétoacétate et de (R)-3-hydroxybutyrate dans le sang du sujet traité augmente pour atteindre 0,1 à 30 mM.

3. Composé pour utilisation telle que revendiquée dans la Revendication 2, **caractérisé en ce que** la concentration totale d'acétoacétate et de (R)-3-hydroxybutyrate dans le sang est comprise entre 0,5 et 15 mM.

4. Composé pour utilisation telle que revendiquée dans la Revendication 2, **caractérisé en ce que** la concentration totale d'acétoacétate et de (R)-3-hydroxybutyrate dans le sang du sujet augmente pour atteindre 1 à 10 mM.

5. Composé pour utilisation telle que revendiquée dans la Revendication 2, **caractérisé en ce que** la concentration totale d'acétoacétate et de (R)-3-hydroxybutyrate dans le sang augmente pour atteindre 3 à 8 mM.

6. Composé pour utilisation telle que revendiquée dans l'une quelconque des revendications précédentes, **caractérisé en ce qu'**il est sous la forme d'une composition pharmaceutique contenant le composé en tant qu'ingrédient actif, conjointement avec des composés diluants, excipients et/ou véhicules.
